# EUROPEAN PATENT SPECIFICATION

(11) **EP 3 205 367 B1**
(45) Date of publication and mention of the grant of the patent: **17.11.2021**
(21) Application number: 17155237.5
(22) Date of filing: 08.02.2017
(51) Int. Cl.: A61M 25/01, A61B 1/005

(54) **STEERABLE CATHETER**
LENKBARER KATHETER
CATHÉTER ORIENTABLE

(30) Priority: 10.02.2016 US 201662293549 P
(43) Date of publication of application: 16.08.2017
(73) Proprietor: Cook Medical Technologies LLC, Bloomington, IN 47404 (US)
(72) Inventor: MELSHEIMER, Jeffry S., Springville, IN Indiana 47642 (US)
(74) Representative: Mathys & Squire

(56) References cited:
- US-A1- 2010 280 449
- US-A1- 2013 096 553
- US-B1- 6 286 514

## Description

### TECHNICAL FIELD

This disclosure relates to elongate medical devices such as sheaths, catheters, scopes, and the like that are configured for medical clinical use, and specifically where it is desired for a portion of the elongate medical device to follow along a curve to match a portion of the anatomy through which the device is desired to be extended, as well as to allow the catheter to deflect in order for the distal tip to be directed in a specific desired direction or orientation, such as to allow for observing or interacting with different parts of the anatomy.

Reference is directed to US 6,286,514 which discloses a computerized imaging system that provides real-time computer control to maintain and adjust the position of the treatment system and/or the position of the patient relative to the treatment system; and also provides real-time computer control of the operation of the treatment system itself.

Reference is further directed to US 2013/0096553 which discloses a deflectable medical device that includes a catheter shaft having a distal end. An ablation electrode may be disposed at the distal end. A deflection mechanism may be coupled to the catheter shaft. The deflection mechanism may include a deflection body and a pull wire coupled to the deflection body. The deflection body may have a longitudinally-extending furrow formed therein. A flex member may be disposed adjacent to the deflection mechanism.

### BRIEF SUMMARY

An aspect of the invention provides a catheter as set out in Claim 1.

A further aspect of the invention provides a method of constructing a catheter as set out in Claim 10.

Advantages of the present disclosure will become more apparent to those skilled in the art from the following description of the preferred embodiments of the disclosure that have been shown and described by way of illustration.

Accordingly, the drawings and description are to be regarded as illustrative in nature and not as restrictive.

### BRIEF DESCRIPTION OF THE DRAWINGS

FIG. 1 is a side view of a catheter with the distal end portion disposed in an arcuate configuration in first direction.
FIG. 2 is the catheter of FIG. 1 with the distal end portion disposed in a longitudinal configuration.
FIG. 3 is the catheter of FIG. 1 with the distal tip portion removed, with the portions of the distal end portion staggered to allow for ease of viewing the various components of the distal end portion.
FIG. 4 is the view of FIG. 3 with the outer sheath disposed proximally of the distal end of the distal end portion.
FIG. 5 is a schematic perspective view of a plurality of aligned and adjacent rigid elements depicting some of the rigid elements (circle A) in pivoted relationships with neighboring rigid elements, and some other rigid elements (circle B) in longitudinally aligned relationships.
FIG. 5a is a perspective view of a plurality of aligned and adjacent rigid elements in a pivoted relationship.
FIG. 5b is the view of the FIG. 5a with the plurality of elements in a longitudinally aligned relationship.
FIG. 6 is a side view of a plurality of aligned and adjacent rigid elements depicting the rigid elements (circle C) with pivoted relationships with neighboring rigid elements.
FIG. 6a is the side view of FIG. 6 depicting the rigid elements aligned along a longitudinal axis.
FIG. 7 is a detail view of circle C from FIG. 6 with the neighboring rigid elements in contact with each other.
FIG. 7a is the detail view of circle C from FIG. 6 with the neighboring rigid elements closely spaced from each other.
FIG. 8 is a detail view of circle D from FIG. 6a with neighboring rigid elements in contact with each other.
FIG. 8a is a detail view of circle D from FIG. 6a with neighboring rigid elements closely spaced from each other.
FIG. 9 is a perspective view of a rigid element usable with catheter of FIG. 1.
FIG. 9a is a side view of a rigid segment.
FIG. 9b is a top view of the rigid element of FIG. 9a.
FIG. 10 is a cross-sectional view of section F—F of FIG. 1, depicting a cross-section of one embodiment of the distal end portion.
FIG. 11 is another cross-sectional view of section F—F of FIG. 1, depicting a cross-section of another embodiment of the distal end portion.
FIG. 12 is yet another cross-sectional view of section F—F of FIG. 1, depicting a cross-section of yet another embodiment of the distal end portion.
FIG. 13 is a cross-sectional view of section G—G of FIG. 1, depicting a representative cross-section of one embodiment of the proximal end portion.
FIG. 14 is an end view of the distal tip portion of the catheter of FIG. 1.

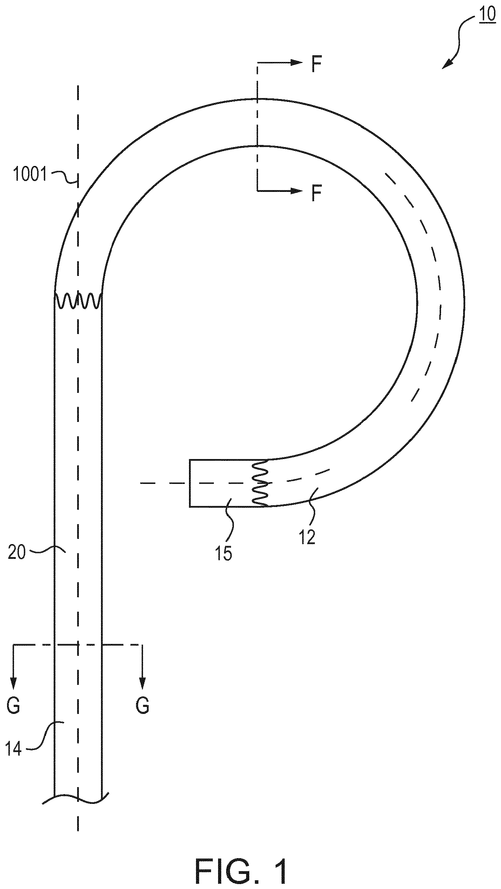

Figures 1-14 depict embodiments of the invention.

### DETAILED DESCRIPTION OF THE DRAWINGS AND THE PRESENTLY PREFERRED EMBODIMENTS

Turning now to FIGs. 1-14, an elongate catheter 10 is provided. The catheter 10 may be used for various clinical medical uses, and may take many forms, such as a catheter, an access sheath, a scope, or the like. For the sake of brevity, the term "catheter" may be used to describe a device with all of these clinical uses, any structural or operational differences with the catheter 10 for various clinical uses will be discussed in detail below.

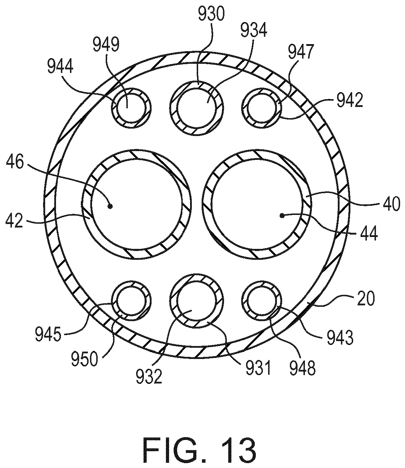

The catheter may extend from a distal tip 15 to a proximal end portion 14. The proximal end portion 14 may include one or more hubs (not shown) that provide convenient portions for accessing or connecting to one or more of the various lumens, discussed below, through the catheter 10, as well as the interacting with other operable portions of the catheter. For example, the catheter 10 may include one or more optical lenses or sensors at or proximate to the distal tip 13, and the catheter may include an output connector or port to allow for interaction with the optical lenses or sensors, such as through an optical fiber or via other communication from the lens or sensor, such as an eye piece for direct viewing, or a connector to pass signal from the optical lens or sensor to a remote viewing means, such as TV or monitor, computer, or the like. Ports may additionally be provided to allow for flushing, suction, illumination, etc. through the catheter 10 and at a location proximate to the distal tip (and/or proximate to another portion of the catheter). Ports may also be provided to allow tools to be provided through one or more lumens of the catheter for use at a location proximate to the distal tip (or another location), such as laser fibers, baskets, forceps, needles, or the like. A schematic cross-sectional view of a potential proximal end portion 14 is depicted in FIG. 13, which shows several lumens 44, 46, 947, 948, 949, 950 (discussed below) therethrough, some of which are configured for pull wires, others for tools, others for a light fiber, suction, aspiration, and the like. One or more of the lumens through the proximal end portion 14 may be formed as a gap in the body 18 of the proximal end portion, or alternatively, one or more of the lumens may be formed through lumens in cannula 50, 54, 942, 932, 934 that extend through the proximal end portion 14 (which may also extend through the distal end portion 12).

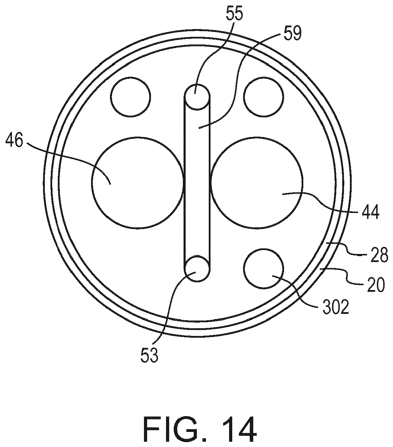

An end view of the distal tip portion 15 is depicted in FIG 14. The distal tip portion may include openings connected with lumens, e.g. 44, 46 that allow communication through and out of the distal tip portion 15 of tools that extend therethrough. Further the distal tip portion 15 may include a lens or sensor 302 that communicates through a light fiber or signal path a view of the environment proximate the distal tip portion 15. Additional lumens, when provided may also communicate through the distal tip portion.

As mentioned herein, the distal ends of the one or more pull wires 53, 55 are fixed with respect to the distal tip portion 15, either fixed directly to the distal tip portion, or fixed to a fastener that is aligned with the distal tip portion. In one embodiment depicted in FIG. 14, the distal ends of the one or both of the pull wires 53, 55 may be mechanically fixed (such as with a fastener, welding, soldering, press fitting, or the like) to the distal tip, such as with the portion of the pull wire that is a continuation of the distal tip of both the first and second pull wires 53, 55. Other methods and structures may be provided to fix the distal ends of the pull wires 53, 55 with respect to the distal tip portion 15 such that the distal ends are fixed with respect to the distal tip portion when one or both of the pull wires 53, 55 are pulled in tension from the proximal end portion 14 of the catheter. In some embodiments, the distal ends of the pull wires 53, 55 may be fixed to a structure within the distal end portion 12, such as a portion of the distal most rigid element 60, discussed below.

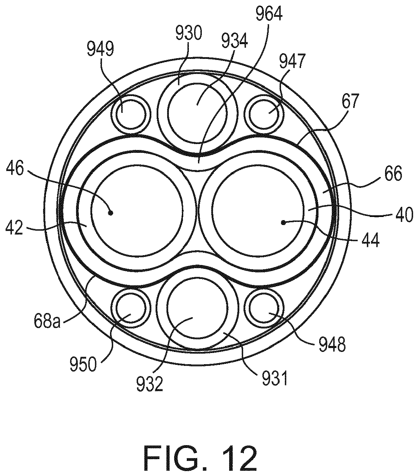

As depicted in FIG. 12, the catheter may include a plurality of dedicated lumens 44, 46 for different tools, as well as one or more dedicated lumens for aspiration and suction 932, 934, lumens for an optical fiber or signal transmission, may be provided as well. In some embodiments, the catheter may include dedicated lumens 947, 948, 949, 950 for pull wires (although in some embodiments only a single lumen for a pull wire is provided, or only a single lumen for a pull wire on each side of the rigid segment, discussed below. Alternatively, the pull wires 53, 55 may extend through a lumen that is also used for other purposes, such as aspiration and suction lumens 932, 934.

The catheter 10 includes a sheath 20 that extends between the distal tip portion 15, through the distal end portion 12, and through the proximal end portion 14. The sheath 20 provides the outer surface of the catheter 10 and is formed from a strong and flexible material.

In some embodiments, the sheath 20 may include multiple coaxial layers, including an outer layer, as well as a woven layer 28 that extends along the entire or a portion of the length of the catheter. The woven layer 28 may consist of braided filaments of Stainless Steel or other medical grade metals, or polymer filaments. The woven layer may be beneficial to provide for added torqueability along the length of the sheath, i.e. to allow a rotation of the proximal end portion 14 of the catheter to transmit the rotational force to the distal tip, rather than causing the catheter to significantly twist along its axis. The woven layer 28 may be formed from a woven mesh, or braid that is formed as a tube and attached to the outer layer of the sheath, such as via co-extrusion of the woven layer with the outer layer. The sheath 20 may be formed separately from the body of the catheter and slid onto the body after the body has been assembled, and retained longitudinally and rotationally in place upon the body, such as by heat shrink lamination, otherwise known as heat shrunk, mechanically crimping, adhesive, or via other methods.

The catheter 10 includes a proximal end portion 14, a distal end portion 12, and a distal tip portion 15. The proximal end portion 14 may be a generally elongate portion that is formed with one or more lumens extending therethrough, as discussed below, and extends from the distal end portion 12 to the hubs (discussed above) at the proximal end of the catheter. The proximal end portion 14 may be configured to extend in a relatively straight orientation along its length with a longitudinal axis 1001 extending therethrough. The proximal end portion 14 may be flexible such that the proximal end portion 14 may be curved at one or more portions along its length without kinking the outer surface or any of the lumens that are disposed through the proximal end portion 14. The proximal end portion 14 may additionally include a steering mechanism that is manipulated by the user to urge the distal end portion 12 to translate between an elongate, relatively straight configuration and an arcuate configuration, discussed above. The proximal end portion 14 may include lumens to allow pull wires, or other structures, to pass therethrough such that the manipulation of the pull wires causes movement of the distal end portion 12 as desired, and the proximal end portion 14 may include one or more mechanisms to allow the user to control the motion of the distal end portion remotely from the proximal end portion 14. The term "wire" is used in this specification and claims for the sake convenience. One of ordinary skill in the art will understand that the term "wire" can include any elongate structure that is capable of being pulled in tension without any substantial elongation, and includes, a metal wire, a chain, a rope, a suture, a flexible cannula, or the like.

The proximal end portion 14 is stiffer than the distal end portion 12 of the catheter such that, as the as the pull wires 53, 55 of the catheter 10 are pulled proximally, the distal end portion 12 curves away from the longitudinal axis 1001 of the proximal end portion 14, while the proximal end portion is maintained relatively straight and aligned with the longitudinal axis.

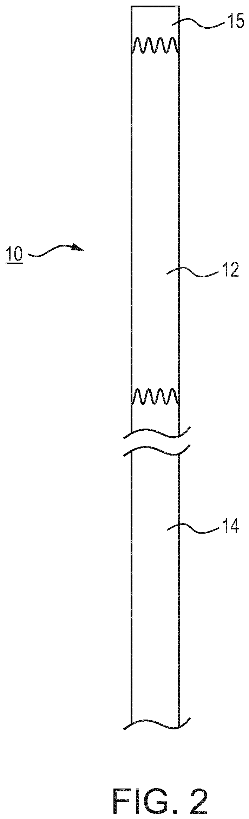

The distal end portion 12 extends from the proximal end portion 12 to the distal tip 15. As discussed in further detail below, the distal end portion 14 is repositionable between a first configuration (FIG. 2) where the distal end portion 12 extends along the longitudinal axis 1001 of the proximal end portion 14, and to one or more arcuate configurations (FIG. 1) where the distal end portion 12 curves arcuately from the longitudinal axis 1001. The distal end portion 12 may be configured to allow the distal end portion 12 to extend from a relatively straight configuration to an arcuate configuration where the distal end portion curves along an arc length of about 270 degrees with respect to the longitudinal axis, as shown in FIG. 1. In other embodiments, the distal end portion 12 may be capable of other arc lengths, such as 235, 180, 135, and 90 degrees, as well as all intermediate positions between these arc lengths.

The distal end portion 12 of the catheter includes a single lumen 40, or in other embodiments two or more lumens 40, 42 that are configured to allow tools or devices to pass through and ultimately to and through apertures in the distal tip portion 15 of the catheter. In addition to lumens for tools, the distal end portion 12 may include dedicated lumens for aspiration/suction, a fiber optic line or visual data transfer, and in some embodiments a dedicated lumen for each pull wire 53, 55, discussed below.

The catheter 10 may include one or more pull wires 53, 55 that extend along the length of the catheter from the proximal end portion 14 and through the distal end portion 12. In some embodiments, distal ends of the pull wires 53, 55 may be fixed to the distal tip portion 15 of the catheter, while in other embodiments, distal ends of the pull wires 53, 55 may be fixed to the distal end portion 12 of the catheter. In embodiments where a single pull wire 53 is provided, the distal end portion 12 of the catheter can bend in a single direction away from the straight configuration when the pull wire 53 is manipulated. In other embodiments where two pull wires 53, 55 are provided, normally on opposite sides of the catheter, the distal end portion 12 can bend in two opposite directions when the opposite pull wires 535, 55 are manipulated. In other embodiments three or more pull wires may be provided. In these embodiments, the pull wires may be provided in pairs that are disposed on opposite sides of the plurality of rigid segments 60, as discussed below. For example, FIGs. 12-13 depict lumens 947, 949 through which a first set of pull wires extend, and lumens 948, 950 through which a second set of pull wires extend. The pull wires are normally positioned on opposite sides of the distal end portion 12 (and through the remainder of the catheter). The pull wires 53, 55, (through their lumens 50, 54, when provided) are preferably disposed proximate to and within the outer sheath 20 of the catheter and disposed outboard of the lumens 40, 42, and outboard of the plurality of enclosed segments 60, discussed below. As can be understood, the placement of the pull wires 53, 55 proximate to the outer sheath 20 allows for additional bending of the distal end portion 12 as the pull wires 53, 55 are pulled in tension toward the proximal end portion 14 of the catheter.

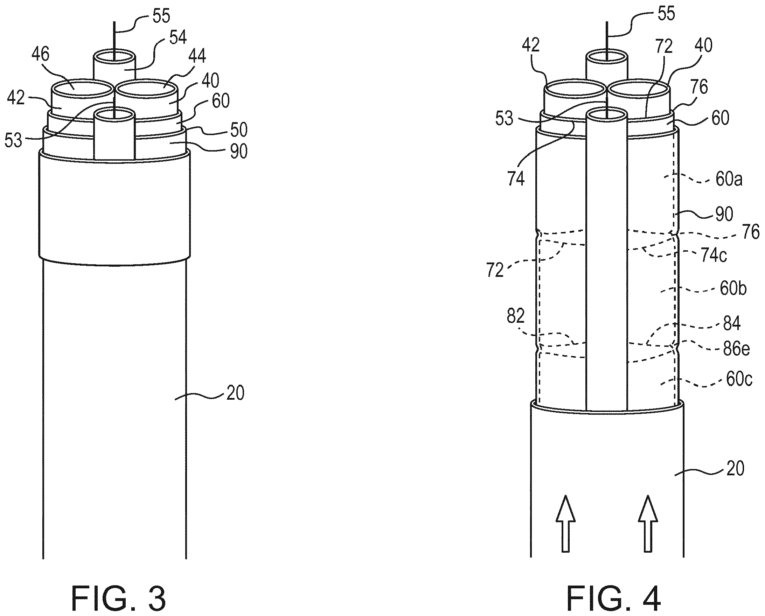

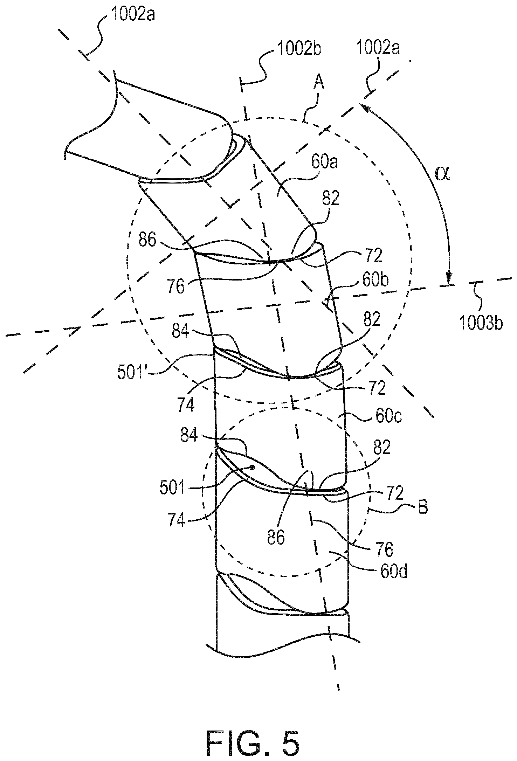

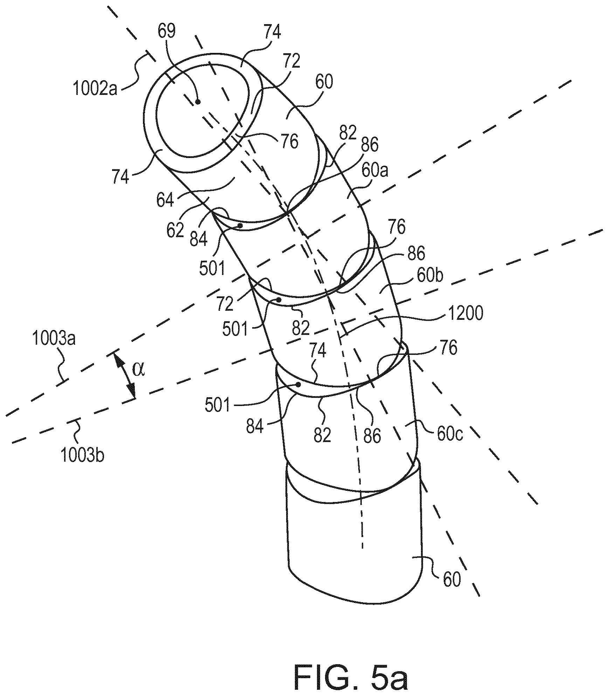

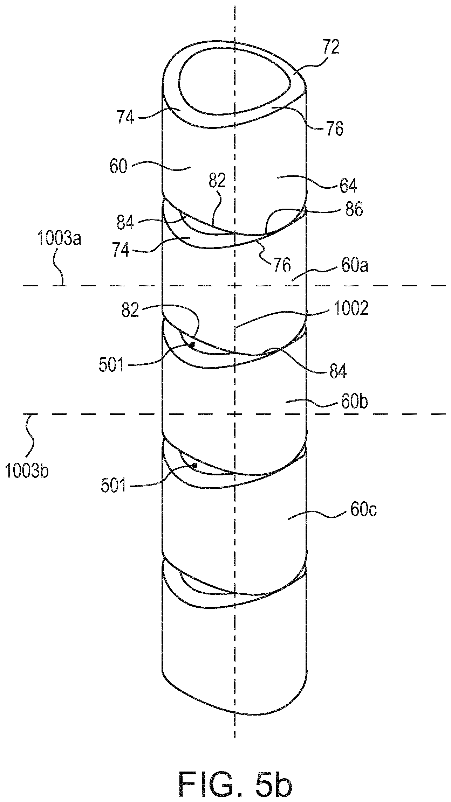

As best shown in FIGs. 4, 5, the distal end portion 12 includes a plurality of rigid segments 60 that are disposed in series along the length of the distal end portion 12. The rigid segments 60 may be machined or molded from a rigid polymer such as PEEK, or cut and formed from pieces of Stainless Steel tubing. When formed from Stainless Steel tubing, the tube may be round or have the desired segment cross-section, such as oval or elliptical. The individual segments may be CNC machined, Laser-Cut or Electrical Discharge Machined (EDM) to provide the necessary contours to each end of the segments 60. The plurality of rigid segments 60 each include a side wall 63 and top and bottom edges 72, 82. The side wall 63 encloses an open cavity 69 that is defined by the side wall 63. The side wall may include opposed side walls 62 and opposed end walls 64 that extend between the ends of each of the first side walls 62. In some embodiments, the side wall 63 is continuous around the entire circumference of the open cavity, while in other embodiments, the side wall 63 may be discontinuous to provide one or more gaps in communication with the open cavity.

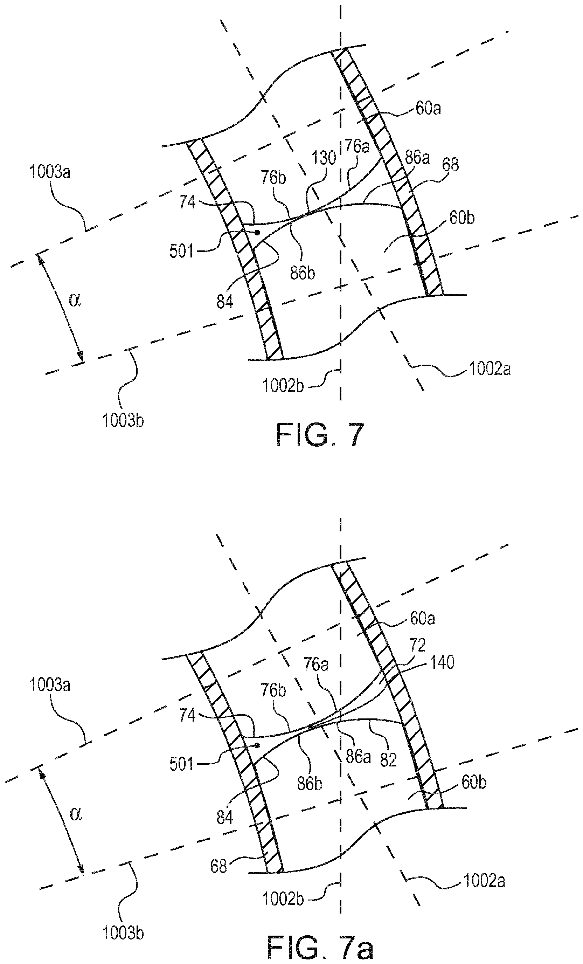

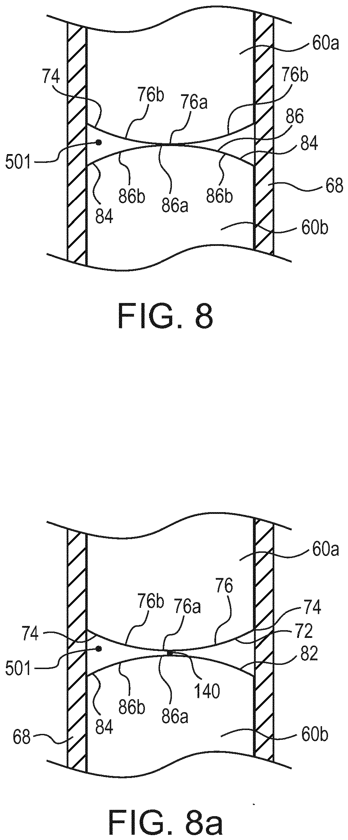

In some embodiments, each of the rigid segments 60 may be formed with the same geometry, while in other embodiments, each of the rigid segments may be formed with the same cross-sectional profile, but may be formed with differing heights. As discussed below, providing rigid segments 60 with differing heights may allow for the distal end portion 12 to bend at different curvatures. For example, a portion of the distal end portion 12 with rigid segments that are relatively shorter than a second portion of the distal end portion 12 that have rigid segments 60 that are longer is able to curve at a larger angle, because the bending occurs at the intersection 130 (FIG. 7, 8) or space 140 (FIG. 7a, 8a) between neighboring rigid segments (because the rigid segments 60 do not significantly bend or deform based upon the level of tension normally imparted upon the pull wires 53, 55 during use of the catheter 10). In still other embodiments, some of the rigid segments may be formed with different geometry. For example, in some embodiments, the most distal rigid segment 60 may be formed without a concave portion upon the top edge 72, and the most proximal rigid segment 60 may be formed without a concave portion upon the bottom edge 82. Alternatively, one of ordinary skill in the art will easily comprehend upon review of the subject application that it is possible to control the amount of possible curvature of the distal end portion 12 by providing aligned rigid segments 60 with different geometries. For example, a section of the distal end portion 12 may be formed to not curve if one or both of the opposed top and bottom edges 72, 82 of adjacent rigid segments 60 do not include concave portions 74, 84. Similarly, the amount of possible relative pivoting (curvature) between adjacent rigid segments 60 may be controlled with the specific geometry of the concave sections 74, 84, with deeper concave sections allowing for greater relative pivoting between adjacent rigid segments 60.

In some embodiments, the profile of the top and bottom edges 72, 82 may be the same for all rigid segments 60, while in other embodiments, the profile of the top and bottom edges 72, 82 may be different, such as with a different depth for the concave portion 74, 84 upon the top and/or bottom edges 72, 82 of different rigid segments. As discussed below and as depicted in FIG. 5, the geometry of the concave portions 74, 84, and the space created between opposed concave potions 74, 84 of adjacent (sometimes in contact) rigid segments defines a void 501 that allows for space for adjacent segments 60 to pivot with respect to each other as one of the pull wires 53, 55 is pulled proximally. A larger void 501 (i.e. with opposed edges that form deeper concave portions 74, 84) allows for adjacent rigid segments to be pivot a greater relative angle than a smaller void 501, because the rigid segments 60 are prevented from further pivoting when the concave portions 74, 84 of adjacent rigid segments 60 contact each other.

In some embodiments, the rigid segments each include a single concave portion 74, 84 on the respective top and bottom edges 72, 82, while in other embodiments each rigid segment 60 includes two concave portions 74, 84 on each respective edge. In some embodiments, the concave portions 74, 84 are disposed on opposite sides of each rigid segment 60, while in other embodiments, the concave portions may be disposed at different relative locations with respect to each other, such as 90 degrees to each other (in embodiments where it is desired to allow the catheter to curve with respect to the longitudinal axis in two directions that are perpendicular to each other. For the sake of simplicity, the position of the two concave portions on opposite sides of the rigid segment 60 from each other, while one of ordinary skill will understand that the concave portions can be positioned at different relative positions with a thorough review and understanding of the subject specification and figures. In some embodiments, three or more concave portions may be provided, with the concave portions evenly distributed about the circumference of the rigid segment, or at other desired spacing relationships.

The top edge 72 and the bottom edge 82 of each rigid segment 60 additionally includes two or more end portions 76, 86 that are disposed between or adjacent to the concave portions 74, 84. In embodiments where the rigid segments 60 include two concave portions 74, 84 on the respective top and bottom edges 72, 82, the end portions 76, 86 are disposed to contact an end of each concave portion. The end portions 76, 86 may include center portions 76a, 86a that extend to the highest (and lowest) longitudinal positions of the edge portions 76, 86. The center portions 76a, 86a may be positioned substantially perpendicular to the longitudinal axis 1002 through the open cavity 69 and parallel to the side walls. The end portions 76, 86 may be curved along their length, with the curve extending through the center portions 76a, 86a. The end portions 76, 86 may be curved along their length with a curve arranged such that adjacent rigid segments 60 have rolling, and in some embodiments continuous contact as the rigid segments 60 pivot with respect to each other. In some embodiments, the end portions 76, 86 may curve toward the concave portions 74, 84 and may be include continuous curvature therebetween. In embodiments where the top and bottom edges 72, 82 each only include a single concave portion 74, 84, the end portion 76, 86 may wrap around a significant portion of the top edge and bottom edge 72, 82 , i.e. for example around about between 200 to 270 degrees of the circumference of the rigid segment, with the concave portion around about 45 to 90 degrees of the circumference, and in some embodiments curved portions 76b, 86b disposed between the center portions 76a, 86a and the concave portion 74, 84.

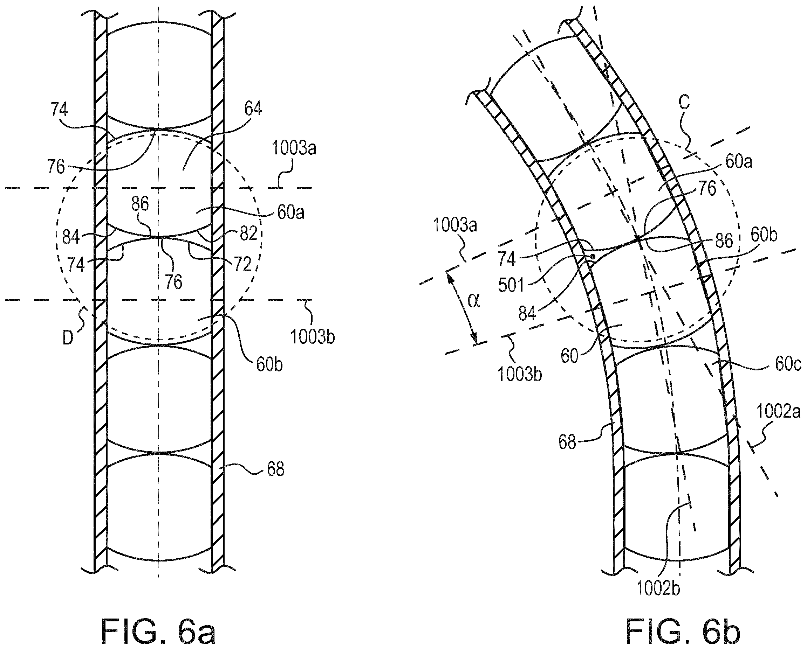

In some embodiments depicted in FIGs. 7 and 8, adjacent aligned rigid segments are positioned such that the end portions 76, 86 of the respective top and bottom edges 72, 82 contact each other, in order for the rigid segments 60 to provide column strength along the length of the distal end portion 12 and also to provide for repeatable curvature of the distal end portion 12, and return to a relatively straight configuration (FIG. 2) as the pull wires 53, 55 are pulled in tension and released. Specifically, when the rigid segments 60 are in their normal longitudinally aligned position (i.e. with the longitudinal axis 1002 through the cavity 69 of each rigid segment 60 collinear), the center portions 76a, 86a contact each other. The center portions 76a, 86a are normally disposed in the middle portion of each end portion 76, 86. As the adjacent rigid segments 60 are urged to pivot with respect to each other (as depicted in circles A in FIGs. 5 and 6, and as shown in FIG. 7), which causes the opposed top and bottom edges 72, 82 to roll with respect to each other, thereby changing the location of contact between the two edges. The movement of the adjacent rigid segments 60 away from the longitudinal position (circle A in FIG. 5 and FIG. 5a) may result in rolling contact of the curved portions 76b, 86b as the adjacent portions further pivot with respect to each other. As can be understood with reference to FIGs. 5 and 6, as the adjacent rigid segments 60 pivot with respect to each other (i.e. to increase the magnitude of the angle α) the space 501 between the opposed concave portions 74, 84 decreases. If the curved portions 74, 84 contact each other, the adjacent segments 60 are prevented from further pivoting with respect to each other.

FIG. 5 is a perspective view of a plurality of rigid segments 60 stacked on each other and depicts a first section, shown in circle A where the adjacent rigid segments are pivoted at an angle α with respect to each other (measured with respect to the angle between an axis 1003 (1003a for rigid segment 60a, and 1003b for rigid segment 60b, each of which are perpendicular to a longitudinal axis 1002a (60a), 1002b (60b)) disposed through the cavity 69 of the respective rigid segment), and a second section, shown in circle B, where the adjacent rigid segments are not pivoted with respect to each other. FIG. 6-8a are side views of a plurality of aligned rigid segments 60a, 60b, 60c, 60d, showing the same relative pivoting of adjacent rigid segments 60 as depicted in FIG. 5. As can be understood and for the sake of convenience and brevity, FIG. 5 is a schematic view intended to depict different possible relative positions of adjacent rigid segments 60 with respect to each other, such as pivoted with respect to each other (circle A) and longitudinally aligned with respect to each other (circle B). One many embodiments, as urged by tension of the corresponding pull wire 53, 55, all of the rigid segments 60 will be urged to pivot with respect to each other, as is depicted in FIG. 5a, such that the overall curve (as depicted by line 1200) through the distal end portion 12 extends along all of the rigid segments 60. As discussed herein, some embodiments, the curve (1200) may be constant along the length of the distal end portion 12 when the rigid segments 60 (depicted as 60a, 60b, 60c in FIG. 5a) each have the same geometry (and the same edge profiles where rigid segments are adjacent to or contact each other), and in other embodiments, the curve may vary along its length where the rigid segments 60 have differing geometry (and/or differing edge profiles for rigid segments that are adjacent or in contact with each other. Similarly, as depicted in FIG. 5b, the distal end portion 12 may be disposed with all of the rigid segments 60 longitudinally aligned when the pull wires are released.

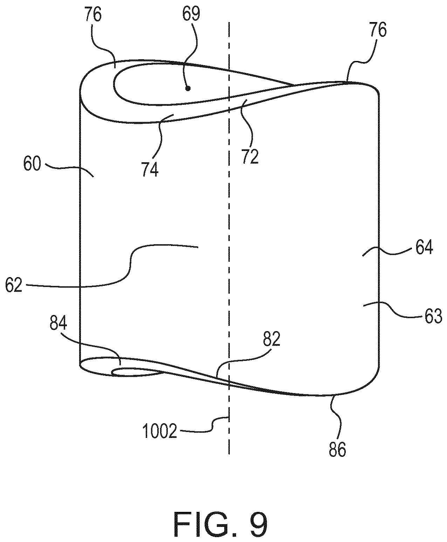

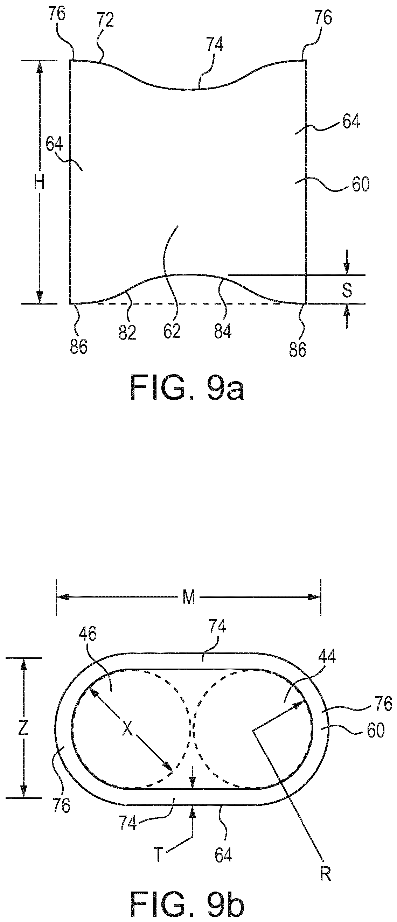

FIGs. 9-9b are detail views of a rigid segment 60, which is configured to allow two lumens 44, 46 to pass through. The lumens, each have a radius of variable R (diameter X) (specifically, when referring to lumens 44, 46 in this context, the radius includes the radius of the lumen 44 itself as well as the wall thickness of the wall of the cannula or tube (40, 42 that forms the lumen, now shown in FIG. 9b, but shown in FIGs. 3 and 4). The thickness of the material forming the rigid segment is depicted with the variable T. In some embodiments, the rigid segment 60 closely surrounds the lumens 40, 44, and therefore the overall width of the rigid segment 60 (variable Z) may be equivalent to (or just slightly larger than) X+2T. The length (variable M) of the rigid segment 60 may be equivalent to (or just slightly larger than 2Z. One of ordinary skill in the art would understand how to size these different variables, specifically based upon the desired radius of the lumen(s) within the rigid segment 60, as well as the necessary wall thickness T for the rigid segment 60 to perform its function of providing substantial column strength along the length of the distal portion, while allowing the distal end portion 12 to pivot along its length (either at a constant arc length along the distal end portion 12 or through varied arcs (when the rigid segments 60 are provided with differing geometries, as discussed herein). One of skill in the art will understand that the necessary thickness T is based upon the strength of the material used for the rigid segment 60, and that the thickness of the rigid segment 60 should be thick enough to ensure that the respective top and bottom edges 72, 82 of adjacent segments 60 maintain contact with each other as the adjacent segments 60 pivot with respect to one another. In some embodiments, the thickness may be 20% of the diameter X of the lumen (40, 44), or may be within a range of between 10% to 30% of the diameter (inclusive of all values within this range).

FIG. 9a depicts a side view of the rigid segment. The height (variable H) the longest portion of the height of the rigid segment 60, normally between opposed end portions 76, 86 of the opposite top and bottom edges 72, 82 of the rigid portion 60. In some embodiments, the height H may be a function of the length of the rigid portion, such as equal to the length M. As discussed elsewhere, differing rigid segments 60 may have different heights H to allow for different curvatures along portions of the distal end portion 12. The amount of scallop of the concave portions 74, 84 is depicted by variable S. As discussed herein, the size of the scallop S effects the possible pivoting between neighboring rigid segments 60. In some embodiments, the scallop S may be a function of another variable of the design of the rigid segment 60, such as S being greater than or equal to 20% of the diameter X of the lumen. Other ranges for S may be used such as between 5% to 30% of the diameter X of the lumen (40, 44), including all values within this range.

In one representative embodiment, the rigid elements 60 may be constructed with a wall thickness of 0.010 inches, and an outer diameter of 0.096 inches (in an embodiment where the rigid elements 60 are cylindrical). The top and bottom edges 72, 86 may be formed with a curvature with a radius of 0.1 inches along its length, or through the center portions 76a, 86a of the end portions 76, 86 and through a portion end portions 76, 86 as the end portions extend toward the concave portions 74, 84. The height of the rigid segments 60 may be 0.01 inches, with the scallop being 0.012 inches (e.g. the vertical distance between the height at the center portion 76a to the lowest point of the concave portion. One of ordinary skill in the art will understand that in some embodiments, the dimensions of the rigid elements (and the remainder of the catheter) may be scaled upward or downward as desired, with limitations due to manufacturability and strength on the low end, and limitations based upon size and weight on the upper end. The specific sizing of components will be readily understood by one of ordinary skill in the art upon a thorough review of the subject specification.

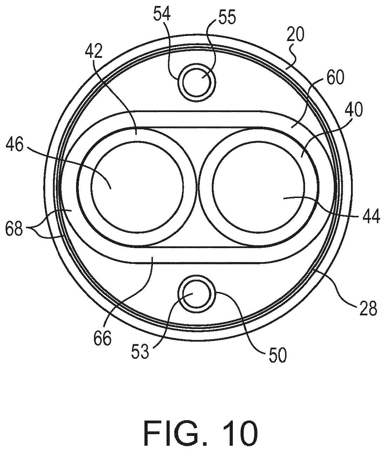

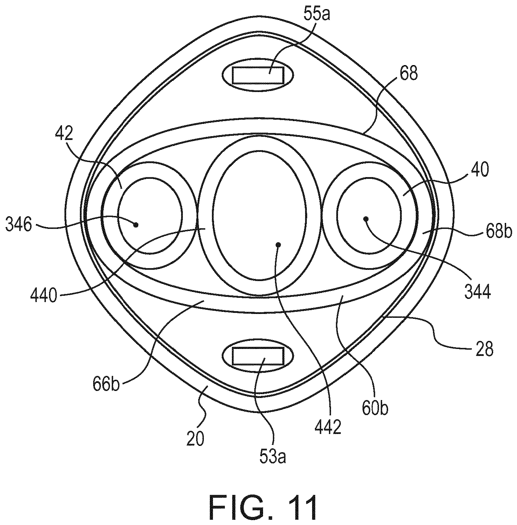

As depicted in FIGs. 9 and 9b, in some embodiments, the rigid segment 60 may have curved end portions 66 and relatively straight side portions 64 that extend between ends of the end portions 66. This design may be beneficial to support and enclose two parallel lumens 40, 44 that are of the same size. In other embodiments, the rigid segments 60 may have a different cross-section. For example, as shown in FIG. 11, the rigid segments 60 may be oval, such as elliptical or other geometries with outwardly curved side portions 64, which allows for a major lumen 444 and one or more minor lumens 344, 346 disposed outboard of the major lumen 444. In other embodiments depicted in FIG. 12, the rigid section 60 may have one or both side portions 64 formed with an indentation 964, which provides for additional space within the sheath 20 but outboard of the rigid segment 60, and causes the rigid element 60 to more closely enclose the inner lumens 40, 44 than with the straight walled embodiment depicted in FIG. 10. The indentation 964 may provide space for a lumen 744 to be disposed outboard of the rigid segment 60 and inboard of the sheath, or at least a lumen 744 with a larger diameter than would be possible with the same size sheath a rigid segment with straight walls. In other embodiments, the rigid segments 60 may be cylindrical in profile, as depicted in FIG. 5a, or may be close to cylindrical with portions of the side walls 62 that include the concave portions 74, 84 upon the edges being formed with a larger radius, or in other embodiments with opposed straight profiles. It will be understood that the catheter 10 may be constructed with rigid segments 60 that are formed with one of the different cross-sectional shapes disclosed herein, which may be chosen based upon the size and number of lumens desired with the catheter or other constraints.

In some embodiments as shown in FIG. 4, FIG. 6, and FIG. 6a, the plurality of aligned rigid segments 60 may be fixed together with an inner sleeve 68. The inner sleeve 68 may be wrapped around the outer surface of the side walls 63 of each rigid segment 60 and may act to maintain the close adjacent alignment (either touching 130 or in close proximity 140) between neighboring rigid segments 60, both when the rigid segments 60 are in longitudinal straight alignment and when they are in curved or pivoted alignment with respect to each other. The inner sleeve may also maintain the desired spacing between adjacent rigid segments 60 as the distal end portion 12 is curved and then returned to the longitudinal position. As discussed further below, the inner sleeve 68 may be heat shrunk to the plurality of rigid segments 60 to prevent relative sliding between the inner sleeve 68 and the various segments 60. In some embodiments, the inner sleeve 68 may include, or a second sleeve 68a may be provided with the inner sleeve 68, which includes a braided layer, which provides torsional strength to the catheter along its length. The braided second sleeve 68a may be in addition to or instead of the braided layer 28 at the outer sleeve 20.

As depicted in FIG. 11, in some embodiments, the one or more pull wires may be formed as flat wires 53a, 55a. In those embodiments the wire lumens 50, 53 may be elliptical or oval to closely surround the cross-sectional geometry of the pull wires 53a, 55a, while in other embodiments, the wire lumens 50, 53 may be circular as depicted in other embodiments.

A possible method of manufacture of the catheter 10 discussed herein is provided, and can be understood with reference to FIGs. 3 and 4. In some embodiments, a proximal end portion 14 that includes the desired lumens (discussed above) is formed, such as by co-extrusion of the body of the proximal end portion along with cannula that defines each desired lumen (such as cannula 40, 42, 50, 54, and the like. The co-extrusion of the proximal end portion 14 is formed such that the cannula extend beyond the distal end of the proximal end portion (which will later incorporate the distal end portion 12 and in some embodiments the distal tip portion15. In other embodiments, the proximal end portion 24 is extruded with the lumens being formed via walls in the body of the proximal end portion, using extrusion techniques to form one or more lumens through the body of a catheter extrusion as are well known in the art and need not be repeated here for the sake of brevity.

Next, in embodiments where the lumens in the proximal end portions are formed without cannula, cannula may be connected to each lumen and extend distally from the lumens at the distal end of the proximal end portion. Alternatively, a body that forms the inner lumens 44, 46 through the distal end portion can be formed (using known techniques) and is aligned with the distal end of the proximal end portion 14, such that the lumens are aligned through the distal end portion 12, and the proximal end portion 14.

Next, a plurality of rigid segments 60 are threaded over the lumens (i.e. the cannula e.g. 40, 44 defining the lumens, or the body of defining the lumens) until the proximal most rigid segments rests upon the distal end of the proximal end portion 14. Additional rigid segments 60 are then individually threaded until all of the desired rigid segments 60 have been threaded. In some embodiments, the rigid segments 60 may be allowed to contact each other at the respective top and bottom edges 72, 82 of the neighboring adjacent rigid segments 60, and specifically the end portions 76, 86 of the those edges. In other embodiments, some or all of the neighboring adjacent rigid segments 60 may be maintained at a spaced relationship from each other, and tooling may be provided, such a comb-like structure with legs that extend between adjacent segments 60 to maintain the spacing. The rigid segments 60 are aligned such that each of the first concave segments 74, 84 (upon the respective top and bottom edges 72, 82) are aligned facing the concave segment on the opposite edge of the adjacent rigid segment, with the first concave segments 74, 84 all in radial alignment with each other along the length of the distal end portion 12. In embodiments, where the top and bottom edges 72, 82 include two or more concave surfaces, then the rigid segments are aligned such that the all of the second concave surfaces 74, 84 (as well as further additional concave surfaces) on the respective top and bottom edges 72, 82 are also in radial alignment and facing each other and in closely adjacent each other.

When all of the desired rigid segments 60 have been threaded over the lumens, a sleeve 68 is threaded over the desired rigid segments 60 and when in place may be fixed to the outer surface of all of the rigid elements 60 (such as by heat shrinking or adhesive, or the like) to closely fit with an maintain the desired longitudinal alignment between neighboring adjacent rigid segments 60. Alternatively, a sheet may be wrapped around the rigid segments 60 and fixed along a seam to form a sleeve, and then heat shrunk or otherwise fixed to the rigid segments 60 to maintain alignment therewith.

Next, a distal tip 15 is fixed to the distal end of the distal end portion 12. The distal tip 15 is aligned such that the appropriate lenses and apertures on the distal tip are aligned with the lumens that extend through the distal end portion 12 and the proximal end portion 14, such as to align a lens on the distal tip with a light fiber or sensor that is provided to view an image proximate to the distal tip 15 of the catheter, and apertures are provided to allow tools and the like (as well as fluids, gas, or suction) items to pass from the lumens and therethrough.

Next, one or more pull wires 53, 55 are fixed with respect to the distal tip 15, such as directly to the distal tip, or to structure within the distal end portion 12 (e.g. to the distal most rigid segment 60) and threaded through the distal end portion 12, and proximal end portion 14 so that it/they may be manipulated by the user from the proximal end portion 14, normally outside of the patient. The pull wires 53, 55 may be threaded through cannula (e.g. 50, 54) or lumens formed in the body of the catheter, or through a combination of the two.

Next an outer sheath is disposed around the length of the catheter 10, at least along the distal end portion 12, and the proximal end portions 14. The outer sheath 20 maybe heat shrunk or otherwise fixed to the components of the distal end portion 12 and/or to the proximal end portion 14.

The invention is defined by the appended claims, which are to be interpreted in accordance with Article 2 of The Protocol on Interpretation of Article 69 EPC.

## Claims

1. An elongate catheter (10), comprising:
an elongate sheath (20) extending between a distal end portion (12) and a proximal end portion (14), and a first lumen that extends through the sheath (20) between the distal end portion (12) and the proximal end portion (14),
the distal end portion (12) extends from the proximal end portion (14) to a distal tip (15) and is configured to be repositionable between a first configuration where the distal end portion (12) extends along a longitudinal axis (1001) of the proximal end portion (14), and a first arcuate configuration where the distal end portion curves arcuately away from a longitudinal axis of the proximal end portion,
the distal end portion (12) comprises a plurality of rigid segments (60) that are disposed in series along the length of the distal end portion (12) within the sheath (20), the plurality of rigid segments (60) each includes a top edge (72), a bottom edge (82), a side portion (64) extending between the top edge (72) and the bottom edge (82), and an inner cavity disposed therethrough, wherein the plurality of rigid segments (60) are disposed such that the top edge (72) of a second rigid segment of the plurality of rigid segments is disposed below the bottom edge of a first rigid segment of the plurality of rigid segments (60), wherein the first rigid segment is disposed closer to the distal tip (15) than the second rigid segment, wherein the plurality of rigid segments (60) are configured such that an amount of possible curvature of the distal end portion (12) can be controlled by providing the plurality of rigid segments (60) with different geometries, and wherein the first lumen extends through the inner cavity of each of the plurality of rigid segments,
further comprising a first wire (53) disposed through the sheath (20) and fixed with respect to the distal tip (15), the first wire extending outboard of each of the plurality of rigid segments (60), wherein the catheter (10) is configured such that a proximal longitudinal force applied to the first wire (53) urges the distal end portion (12) toward the first arcuate configuration, wherein two or more neighboring rigid segments pivot with respect to each other as the distal end repositions between the first configuration and the first arcuate configuration.

2. The catheter (10) of claim 1, wherein the top edge (72) of the second rigid segment includes a first top concave portion and the bottom edge (82) of the first rigid segment establishes a first bottom concave portion, preferably wherein the plurality of rigid segments (60) are disposed such that the first top concave portion of the top edge of the second rigid segment is aligned with the first bottom concave portion of the bottom edge (82) of the first rigid segment disposed distally of the second of the plurality of rigid segments (60), preferably wherein the first wire (53) is disposed proximate to the alignment between the at least one top concave portion of the top edge (72) of the second rigid segment and the at least one bottom concave portion of the bottom edge of the first rigid segment.

3. The catheter (10) of claim 2, wherein the top edge (72) of each of the plurality of rigid segments (60) includes a first top concave portion and the bottom edge (82) of each of the plurality of rigid segments (60) includes a first bottom concave portion, preferably wherein the top edge (72) of the second rigid segment further comprises a second top concave portion that is disposed on an opposite side of the top edge (72) from the first top concave portion, wherein the bottom edge (82) of the first rigid segment further comprises a second bottom concave portion that is disposed on an opposite side of the bottom edge (82) from the first bottom concave portion.

4. The catheter (10) of any one of claims 2-3, wherein the top edge (72) of the second rigid segment includes opposed end portions (76, 86) in connection with respective opposite ends of the first top concave portion, and the bottom edge (82) of the first rigid segment includes opposed end portions (76, 86) in connection with the respective opposite ends of the first bottom concave portion, wherein the opposed end portions of the top edge of the second member and the opposed end portions of the bottom edge of the first member are aligned with each other and adjacent to each other, preferably wherein the opposed end portions (76, 86) of the top edge of the second rigid segment and the opposed end portions of the bottom edge of the first rigid segment make rolling contact with each other as the distal end portion (12) is repositioned between the first orientation and the first arcuate configuration.

5. The catheter (10) of any one of claims 1-4, further comprising a second wire (55) disposed through the sheath (20) and fixed with respect to the distal tip (15) of the catheter (10), the second wire (55) extending outboard of each of the plurality of rigid segments (60) and disposed on an opposite side of the plurality of rigid segments (60) as the first wire (53), wherein the distal end portion (12) is repositionable from the first configuration to a second arcuate configuration where the distal end portion (12) curves arcuately away from the longitudinal direction (1001) in an opposite direction as the curvature when in the first arcuate configuration, wherein the distal end portion (12) repositions toward the second arcuate configuration when a proximal longitudinal force is applied to the second wire (55).

6. The catheter (10) of any one of claims 1-5, further comprising a sleeve (68) disposed around each of the plurality of rigid segments (60), wherein the sleeve (68) maintains the relative longitudinal position (1001) of each of the plurality of elongate members, wherein the first wire (53) is disposed outboard of the sleeve (68), preferably wherein the sleeve (68) is heat shrunk around the plurality of rigid segments (60).

7. The catheter (10) of any one of claims 1-6, further comprising a second lumen extending between the distal end portion (12) and the proximal end portion (14) and disposed outboard of the plurality of rigid segments (60).

8. The catheter (10) of any one of claims 1-7, wherein the opposed bottom and top edges (82, 72) of neighboring rigid segments make rolling contact with respect to each other as the distal end portion (12) repositions between the first orientation and the first arcuate configuration.

9. The catheter (10) of claim 1, wherein at least one of the plurality of rigid segments (60) does not include one or both of a concave portion upon the top edge (72) and a concave portion upon the bottom edge (82).

10. A method of constructing an elongate catheter, comprising:
forming a distal end portion (12) that includes inner lumens (40,44) extending from the distal end portion (12) to a proximal end portion (14), the distal end portion (12) comprising a distal tip portion (15) at a distal end of the distal end portion (12);
forming a plurality of rigid segments (60) with different geometries, each of the plurality of rigid segments (60) includes a top edge (72), a bottom edge (82), and a side portion (64) extending between the top edge (72) and the bottom edge (82);
sliding the plurality of rigid segments (60) within the distal end portion (12)along a portion of the length of the inner lumens (40,44) proximate to and proximal of the distal tip portion (15), such that the inner lumens (40,44) pass through the plurality of rigid segments (60) and disposing the plurality of rigid segments (60) in close proximity to each other such that the top edge (72) of a second segment of the plurality of rigid segments is proximate to and facing the bottom edge (82) of a first segment of the plurality of rigid segments,
disposing a sleeve (68) around the plurality of rigid segments (60),
aligning a wire (53) from the distal end portion (12) of the elongate catheter (10) and to a proximal tip of the proximal end portion (14) of the elongate catheter (10), the wire (53) disposed outboard of each of the plurality of rigid segments,
disposing an outer sheath (20) around the sleeve and the wire,
and fixing a distal portion of the wire (53) with respect to the distal tip portion of the elongate catheter (10).

11. The method of claim 10, further comprising aligning the plurality of rigid segments (60) such that an amount of possible curvature of the distal end portion (12) can be controlled by providing the plurality of rigid segments (60) with different geometries,
wherein the top edge (72) of the second rigid segment includes a first top concave portion and the bottom edge (82) of the first rigid segment of the plurality of rigid segments (60) includes a first bottom concave portion, wherein the first and second rigid segments are aligned such that the top and bottom concave portions are proximate to and face each other.

12. The method of claim 11, wherein the wire (53) is disposed outboard of and proximate to the first top concave portion and the first bottom concave portion of the respective second and first rigid segments.

13. The method of any one of claims 11-12, wherein the top edge (72) of the second rigid segment further comprises a second top concave portion that is disposed on an opposite side of the top edge (72) from the first top concave portion, and wherein the bottom edge (82) of the first rigid segment further comprises a second bottom concave portion that is disposed on an opposite side of the bottom edge (82) from the first bottom concave portion, and
aligning a second wire (55) from the distal tip of the distal end portion (12) of the elongate catheter (10) and to a proximal tip of the proximal end portion (14) of the elongate catheter (10), wherein the second wire (55) is disposed outboard of the plurality of rigid segments (60) and proximate to the second top concave portion and the second bottom concave portion.

14. The method of claim 11, further comprising positioning the plurality of rigid segments (60) such that the top edge (72) of the second rigid segment is in contact with the bottom edge (82) of the first rigid segment positioned adjacent to the second rigid segment and distally of the second rigid segment.

15. The method of any one of claims 11-14, further comprising heat shrinking the sheath (20) disposed around the plurality of rigid segments (60).

## Patentansprüche

1. Länglicher Katheter (10), umfassend:
eine längliche Hülse (20), die sich zwischen einem distalen Endabschnitt (12) und einem proximalen Endabschnitt (14) erstreckt, und ein erstes Lumen, das sich zwischen dem distalen Endabschnitt (12) und dem proximalen Endabschnitt (14) durch die Hülse (20) erstreckt,
wobei sich der distale Endabschnitt (12) von dem proximalen Endabschnitt (14) zu einer distalen Spitze (15) erstreckt und zwischen einer ersten Konfiguration, in der sich der distale Endabschnitt (12) entlang einer Längsachse (1001) des proximalen Endabschnitts (14) erstreckt, und einer ersten bogenförmigen Konfiguration, in der sich der distale Endabschnitt bogenförmig von einer Längsachse des proximalen Endabschnitts weg krümmt, umpositionierbar ausgestaltet ist,
wobei der distale Endabschnitt (12) eine Vielzahl von starren Segmenten (60), die in Reihe entlang der Länge des distalen Endabschnitts (12) in der Hülse (20) angeordnet sind, umfasst, wobei die Vielzahl von starren Segmenten (60) jeweils einen oberen Rand (72), einen unteren Rand (82), einen Seitenabschnitt (64), der sich zwischen dem oberen Rand (72) und dem unteren Rand (82) erstreckt, und einen dort hindurch angeordneten inneren Hohlraum aufweisen, wobei die Vielzahl von starren Segmenten (60) so angeordnet sind, dass der obere Rand (72) eines zweiten starren Segments der Vielzahl von starren Segmenten unter dem unteren Rand eines ersten starren Segments der Vielzahl von starren Segmenten (60) angeordnet ist, wobei das erste starre Segment näher der distalen Spitze (15) angeordnet ist als das zweite starre Segment, wobei die Vielzahl von starren Segmenten (60) so ausgestaltet sind, dass ein Ausmaß an möglicher Krümmung des distalen Endabschnitts (12) dadurch gesteuert werden kann, dass die Vielzahl von starren Segmenten (60) mit unterschiedlichen Geometrien versehen werden, und wobei sich das erste Lumen durch den inneren Hohlraum jedes der Vielzahl von starren Segmenten erstreckt,
ferner umfassend einen ersten Draht (53), der durch die Hülse (20) angeordnet und bezüglich der distalen Spitze (15) fixiert ist, wobei sich der erste Draht außerhalb jedes der Vielzahl von starren Segmenten (60) erstreckt, wobei der Katheter (10) so ausgestaltet ist, dass der distale Endabschnitt (12) durch eine auf den ersten Draht (53) ausgeübte proximale Längskraft zu der ersten bogenförmigen Konfiguration hin gedrängt wird, wobei zwei oder mehr benachbarte starre Segmente bei Umpositionierung des distalen Endes zwischen der ersten Konfiguration und der ersten bogenförmigen Konfiguration bezüglich einander schwenken.

2. Katheter (10) nach Anspruch 1, wobei der obere Rand (72) des zweiten starren Segments einen ersten oberen konkaven Abschnitt aufweist und der untere Rand (82) des ersten starren Segments einen ersten unteren konkaven Abschnitt aufweist, vorzugsweise wobei die Vielzahl von starren Segmenten (60) so angeordnet sind, dass der erste obere konkave Abschnitt des oberen Rands des zweiten starren Segments auf den ersten unteren konkaven Abschnitt des unteren Rands (82) des ersten starren Segments, das distal zu dem zweiten der Vielzahl von starren Segmenten (60) angeordnet ist, ausgerichtet ist, vorzugsweise wobei der erste Draht (53) nahe der Ausrichtung zwischen dem mindestens einen oberen konkaven Abschnitt des oberen Rands (72) des zweiten starren Segments und dem mindestens einen unteren konkaven Abschnitt des unteren Endes des ersten starren Segments angeordnet ist.

3. Katheter (10) nach Anspruch 2, wobei der obere Rand (72) jedes der Vielzahl von starren Segmenten (60) einen ersten oberen konkaven Abschnitt aufweist und der untere Rand (82) jedes der Vielzahl von starren Segmenten (60) einen ersten unteren konkaven Abschnitt aufweist, vorzugsweise wobei der obere Rand (72) des zweiten starren Segments ferner einen zweiten konkaven Abschnitt umfasst, der an einer dem ersten oberen konkaven Abschnitt gegenüberliegenden Seite des oberen Rands (72) angeordnet ist, wobei der untere Rand (82) des ersten starren Segments ferner einen zweiten unteren konkaven Abschnitt umfasst, der an einer dem ersten unteren konkaven Abschnitt gegenüberliegenden Seite des unteren Rands (82) angeordnet ist.

4. Katheter (10) nach einem der Ansprüche 2 - 3, wobei der obere Rand (72) des zweiten starren Segments gegenüberliegende Endabschnitte (76, 86) in Verbindung mit jeweiligen gegenüberliegenden Enden des ersten oberen konkaven Abschnitts aufweist und der untere Rand (82) des ersten starren Segments gegenüberliegende Endabschnitte (76, 86) in Verbindung mit den jeweiligen gegenüberliegenden Enden des ersten unteren konkaven Abschnitts aufweist, wobei die gegenüberliegenden Endabschnitte des oberen Rands des zweiten Glieds und die gegenüberliegenden Endabschnitte des unteren Rands des ersten Glieds aufeinander und benachbart zueinander ausgerichtet sind, vorzugsweise wobei die gegenüberliegenden Endabschnitte (76, 86) des oberen Rands des zweiten starren Segments und die gegenüberliegenden Endabschnitte des unteren Rands des ersten starren Segments bei Umpositionierung des distalen Endabschnitts (12) zwischen der ersten Ausrichtung und der ersten bogenförmigen Konfiguration rollend miteinander in Kontakt kommen.

5. Katheter (10) nach einem der Ansprüche 1 - 4, ferner umfassend einen zweiten Draht (55), der durch die Hülse (20) angeordnet und bezüglich der distalen Spitze (15) des Katheters (10) fixiert ist, wobei sich der zweite Draht (55) außerhalb jedes der Vielzahl von starren Segmenten (60) erstreckt und bezüglich des ersten Drahts (53) an einer gegenüberliegenden Seite der Vielzahl von starren Segmenten (60) angeordnet ist, wobei der distale Endabschnitt (12) aus der ersten Konfiguration in eine zweite bogenförmige Konfiguration umpositionierbar ist, in der sich der distale Endabschnitt (12) bogenförmig von der Längsrichtung (1001) weg in eine Richtung krümmt, die der der Krümmung in der ersten bogenförmigen Konfiguration entgegengesetzt ist, wobei sich der distale Endabschnitt (12) zu der zweiten bogenförmigen Konfiguration hin umpositioniert, wenn eine proximale Längskraft auf den zweiten Draht (55) ausgeübt wird.

6. Katheter (10) nach einem der Ansprüche 1 - 5, ferner umfassend eine um jedes der Vielzahl von starren Segmenten (60) angeordnete Hülle (68), wobei die Hülle (68) die relative Längsposition (1001) jedes der Vielzahl von länglichen Gliedern aufrechterhält, wobei der erste Draht (53) außerhalb der Hülle (68) angeordnet ist, vorzugsweise wobei die Hülle (68) um die Vielzahl von starren Segmenten (60) herum wärmegeschrumpft ist.

7. Katheter (10) nach einem der Ansprüche 1 - 6, ferner umfassend ein zweites Lumen, das sich zwischen dem distalen Endabschnitt (12) und dem proximalen Endabschnitt (14) erstreckt und außerhalb der Vielzahl von starren Segmenten (60) angeordnet ist.

8. Katheter (10) nach einem der Ansprüche 1 - 7, wobei der untere und der obere gegenüberliegende Rand (82, 72) benachbarter starrer Segmente bei Umpositionierung des distalen Endabschnitts (12) zwischen der ersten Ausrichtung und der ersten bogenförmigen Konfiguration rollend miteinander in Kontakt kommen.

9. Katheter (10) nach Anspruch 1, wobei mindestens eines der Vielzahl von starren Segmenten (60) keinen konkaven Abschnitt an dem oberen Rand (72) oder keinen konkaven Abschnitt an dem unteren Rand (82) oder weder einen konkaven Abschnitt an dem oberen Rand (72) noch einen konkaven Abschnitt an dem unteren Rand (82) aufweist.

10. Verfahren zur Herstellung eines länglichen Katheters, umfassend:
Bilden eines distalen Endabschnitts (12), der innere Lumen (40, 44) aufweist, die sich von dem distalen Endabschnitt (12) zu einem proximalen Endabschnitt (14) erstrecken, wobei der distale Endabschnitt (12) einen distalen Spitzenabschnitt (15) an einem distalen Ende des distalen Endabschnitts (12) umfasst,
Bilden einer Vielzahl von starren Segmenten (60) mit unterschiedlichen Geometrien, wobei jedes der Vielzahl von starren Segmenten (60) einen oberen Rand (72), einen unteren Rand (82) und einen sich zwischen dem oberen Rand (72) und dem unteren Rand (82) erstreckenden Seitenabschnitt (64) aufweist,
Schieben der Vielzahl von starren Segmenten (60) in dem distalen Endabschnitt (12) entlang eines Abschnitts der Länge der inneren Lumen (40, 44) in der Nähe des und proximal zu dem distalen Spitzenabschnitt (15), so dass die inneren Lumen (40, 44) durch die Vielzahl von starren Segmenten (60) gehen, und Anordnen der Vielzahl von starren Segmenten (60) in nächster Nähe zueinander, so dass der obere Rand (72) eines zweiten Segments der Vielzahl von starren Segmenten nahe dem unteren Rand (82) eines ersten Segments der Vielzahl von starren Segmenten liegt und diesem zugewandt ist,
Anordnen einer Hülle (68) um die Vielzahl von starren Segmenten (60),
Ausrichten eines Drahts (53) von dem distalen Endabschnitt (12) des länglichen Katheters (10) und zu einer proximalen Spitze des proximalen Endabschnitts (14) des länglichen Katheters (10), wobei der Draht (53) außerhalb jedes der Vielzahl von starren Segmenten angeordnet ist,
Anordnen einer äußeren Hülse (20) um die Hülle und den Draht,
und Fixieren eines distalen Abschnitts des Drahts (53) bezüglich dem distalen Spitzenabschnitt des länglichen Katheters (10).

11. Verfahren nach Anspruch 10, ferner umfassend derartiges Ausrichten der Vielzahl von starren Segmenten (60), dass ein Ausmaß an möglicher Krümmung des distalen Endabschnitts (12) dadurch gesteuert werden kann, dass die Vielzahl von starren Segmenten (60) mit unterschiedlichen Geometrien versehen werden,
wobei der obere Rand (72) des zweiten starren Segments einen ersten oberen konkaven Abschnitt aufweist und der untere Rand (82) des ersten starren Segments der Vielzahl von starren Segmenten (60) einen ersten unteren konkaven Abschnitt aufweist, wobei das erste und das zweite starre Segment so ausgerichtet sind, dass der obere und der untere konkave Abschnitt nahe beieinander liegen und einander zugewandt sind.

12. Verfahren nach Anspruch 11, wobei der Draht (53) außerhalb des ersten oberen konkaven Abschnitts und des ersten unteren konkaven Abschnitts des jeweiligen zweiten und ersten starren Segments und nahe bei diesen liegt.

13. Verfahren nach einem der Ansprüche 11 - 12, wobei der obere Rand (72) des zweiten starren Segments ferner einen zweiten oberen konkaven Abschnitt umfasst, der an einer dem ersten oberen konkaven Abschnitt gegenüberliegenden Seite des oberen Rands (72) angeordnet ist und wobei der untere Rand (82) des ersten starren Segments ferner einen zweiten unteren konkaven Abschnitt umfasst, der an einer dem ersten unteren konkaven Abschnitt gegenüberliegenden Seite des unteren Rands (82) angeordnet ist, und
Ausrichten eines zweiten Drahts (55) von der distalen Spitze des distalen Endabschnitts (12) des länglichen Katheters (10) und zu einer proximalen Spitze des proximalen Endabschnitts (14) des länglichen Katheters (10), wobei der zweite Draht (55) außerhalb der Vielzahl von starren Segmenten (60) und nahe dem zweiten oberen konkaven Abschnitt und dem zweiten unteren konkaven Abschnitt angeordnet ist.

14. Verfahren nach Anspruch 11, ferner umfassend derartiges Positionieren der Vielzahl von starren Segmenten (60), dass der obere Rand (72) des zweiten starren Segments in Kontakt mit dem unteren Rand (82) des ersten starren Segments steht, das dem zweiten starren Segment benachbart und distal von dem zweiten starren Segment positioniert ist.

15. Verfahren nach einem der Ansprüche 11 - 14, ferner umfassend Heißschrumpfen der um die Vielzahl von starren Segmenten (60) herum angeordneten Hülse (20).

## Revendications

1. Cathéter allongé (10), comprenant :
une gaine allongée (20) s'étendant entre une partie d'extrémité distale (12) et une partie d'extrémité proximale (14), et une première lumière qui s'étend à travers la gaine (20) entre la partie d'extrémité distale (12) et la partie d'extrémité proximale (14),
la partie d'extrémité distale (12) s'étend à partir de la partie d'extrémité proximale (14) jusqu'à une pointe distale (15) et est configurée pour être repositionnable entre une première configuration dans laquelle la partie d'extrémité distale (12) s'étend le long d'un axe longitudinal (1001) de la partie d'extrémité proximale (14), et une première configuration arquée dans laquelle la partie d'extrémité distale se courbe de manière arquée à l'opposé d'un axe longitudinal de la partie d'extrémité proximale,
la partie d'extrémité distale (12) comprend une pluralité de segments rigides (60) qui sont disposés en série sur la longueur de la partie d'extrémité distale (12) à l'intérieur de la gaine (20), la pluralité de segments rigides (60) comprennent chacun un bord supérieur (72), un bord inférieur (82), une partie latérale (64) s'étendant entre le bord supérieur (72) et le bord inférieur (82), et une cavité interne disposée à travers celle-ci, la pluralité de segments rigides (60) étant disposés de telle sorte que le bord supérieur (72) d'un second segment rigide de la pluralité de segments rigides est disposé en dessous du bord inférieur d'un premier segment rigide de la pluralité de segments rigides (60), le premier segment rigide étant disposé plus près de la pointe distale (15) que le second segment rigide, la pluralité de segments rigides (60) étant configurés de telle sorte qu'une quantité de courbure possible de la partie d'extrémité distale (12) peut être commandée en fournissant la pluralité de segments rigides (60) avec différentes géométries, et la première lumière s'étendant à travers la cavité interne de chacun de la pluralité de segments rigides,
comprenant en outre un premier fil (53) disposé à travers la gaine (20) et fixé par rapport à la pointe distale (15), le premier fil s'étendant à l'extérieur de chacun de la pluralité de segments rigides (60), le cathéter (10) étant configuré de telle sorte qu'une force longitudinale proximale appliquée au premier fil (53) entraîne la partie d'extrémité distale (12) vers la première configuration arquée, deux ou plus de deux segments rigides voisins pivotant les uns par rapport aux autres lorsque l'extrémité distale se repositionne entre la première configuration et la première configuration arquée.

2. Cathéter (10) selon la revendication 1, le bord supérieur (72) du second segment rigide comprenant une première partie concave supérieure et le bord inférieur (82) du premier segment rigide établissant une première partie concave inférieure, de préférence la pluralité de segments rigides (60) étant disposés de telle sorte que la première partie concave supérieure du bord supérieur du second segment rigide est alignée avec la première partie concave inférieure du bord inférieur (82) du premier segment rigide disposé distalement par rapport au second de la pluralité de segments rigides (60), de préférence le premier fil (53) étant disposé à proximité de l'alignement entre l'au moins une partie concave supérieure du bord supérieur (72) du second segment rigide et l'au moins une partie concave inférieure du bord inférieur du premier segment rigide.

3. Cathéter (10) selon la revendication 2, le bord supérieur (72) de chacun de la pluralité de segments rigides (60) comprenant une première partie concave supérieure et le bord inférieur (82) de chacun de la pluralité de segments rigides (60) comprenant une première partie concave inférieure, de préférence le bord supérieur (72) du second segment rigide comprenant en outre une seconde partie concave supérieure qui est disposée sur un côté opposé du bord supérieur (72) à partir de la première partie concave supérieure, le bord inférieur (82) du premier segment rigide comprenant en outre une seconde partie concave inférieure qui est disposée sur un côté opposé du bord inférieur (82) à partir de la première partie concave inférieure.

4. Cathéter (10) selon l'une quelconque des revendications 2 et 3, le bord supérieur (72) du second segment rigide comprenant des parties d'extrémité opposées (76, 86) en liaison avec des extrémités opposées respectives de la première partie concave supérieure, et le bord inférieur (82) du premier segment rigide comprenant des parties d'extrémité opposées (76, 86) en liaison avec les extrémités opposées respectives de la première partie concave inférieure, les parties d'extrémité opposées du bord supérieur du second élément et les parties d'extrémité opposées du bord inférieur du premier élément étant alignées les unes avec les autres et adjacentes les unes aux autres, de préférence les parties d'extrémité opposées (76, 86) du bord supérieur du second segment rigide et les parties d'extrémité opposées du bord inférieur du premier segment rigide venant en contact de roulement les unes avec les autres lorsque la partie d'extrémité distale (12) est repositionnée entre la première orientation et la première configuration arquée.

5. Cathéter (10) selon l'une quelconque des revendications 1 à 4, comprenant en outre un second fil (55) disposé à travers la gaine (20) et fixé par rapport à la pointe distale (15) du cathéter (10), le second fil (55) s'étendant à l'extérieur de chacun de la pluralité de segments rigides (60) et étant disposé sur un côté opposé de la pluralité de segments rigides (60) en tant que premier fil (53), la partie d'extrémité distale (12) pouvant être repositionnée à partir de la première configuration jusqu'à une seconde configuration arquée dans laquelle la partie d'extrémité distale (12) se courbe de manière arquée à l'opposé de la direction longitudinale (1001) dans une direction opposée à la courbure lorsqu'elle est dans la première configuration arquée, la partie d'extrémité distale (12) se repositionnant vers la seconde configuration arquée lorsqu'une force longitudinale proximale est appliquée au second fil (55).

6. Cathéter (10) selon l'une quelconque des revendications 1 à 5, comprenant en outre un manchon (68) disposé autour de chacun de la pluralité de segments rigides (60), le manchon (68) maintenant la position longitudinale relative (1001) de chacun de la pluralité d'éléments allongés, le premier fil (53) étant disposé à l'extérieur du manchon (68), de préférence le manchon (68) étant thermorétracté autour de la pluralité de segments rigides (60).

7. Cathéter (10) selon l'une quelconque des revendications 1 à 6, comprenant en outre une seconde lumière s'étendant entre la partie d'extrémité distale (12) et la partie d'extrémité proximale (14) et disposée à l'extérieur de la pluralité de segments rigides (60).

8. Cathéter (10) selon l'une quelconque des revendications 1 à 7, les bords inférieur et supérieur opposés (82, 72) de segments rigides voisins venant en contact de roulement l'un par rapport à l'autre lorsque la partie d'extrémité distale (12) se repositionne entre la première orientation et la première configuration arquée.

9. Cathéter (10) selon la revendication 1, au moins l'un de la pluralité de segments rigides (60) ne comprenant pas l'une ou les deux d'une partie concave sur le bord supérieur (72) et d'une partie concave sur le bord inférieur (82).

10. Procédé de construction d'un cathéter allongé, comprenant :
la formation d'une partie d'extrémité distale (12) qui comprend des lumières internes (40, 44) s'étendant à partir de la partie d'extrémité distale (12) jusqu'à une partie d'extrémité proximale (14), la partie d'extrémité distale (12) comprenant une partie de pointe distale (15) au niveau d'une extrémité distale de la partie d'extrémité distale (12) ;
la formation d'une pluralité de segments rigides (60) avec différentes géométries, chacun de la pluralité de segments rigides (60) comprend un bord supérieur (72), un bord inférieur (82), et une partie latérale (64) s'étendant entre le bord supérieur (72) et le bord inférieur (82) ;
le coulissement de la pluralité de segments rigides (60) à l'intérieur de la partie d'extrémité distale (12) le long d'une partie de la longueur des lumières internes (40, 44) à proximité de et de façon proximale à la partie de pointe distale (15), de telle sorte que les lumières internes (40, 44) passent à travers la pluralité de segments rigides (60), et la disposition de la pluralité de segments rigides (60) à proximité immédiate les uns des autres de telle sorte que le bord supérieur (72) d'un second segment de la pluralité de segments rigides est à proximité du et orienté vers le bord inférieur (82) d'un premier segment de la pluralité de segments rigides,
la disposition d'un manchon (68) autour de la pluralité de segments rigides (60),
l'alignement d'un fil (53) à partir de la partie d'extrémité distale (12) du cathéter allongé (10) et jusqu'à une pointe proximale de la partie d'extrémité proximale (14) du cathéter allongé (10), le fil (53) étant disposé à l'extérieur de chacun de la pluralité de segments rigides,
la disposition d'une gaine extérieure (20) autour du manchon et du fil,
et la fixation d'une partie distale du fil (53) par rapport à la partie de pointe distale du cathéter allongé (10) .

11. Procédé selon la revendication 10, comprenant en outre l'alignement de la pluralité de segments rigides (60) de telle sorte qu'une quantité de courbure possible de la partie d'extrémité distale (12) peut être commandée en fournissant la pluralité de segments rigides (60) avec différentes géométries,
le bord supérieur (72) du second segment rigide comprenant une première partie concave supérieure et le bord inférieur (82) du premier segment rigide de la pluralité de segments rigides (60) comprenant une première partie concave inférieure, les premier et second segments rigides étant alignés de telle sorte que les parties concaves supérieure et inférieure sont à proximité et se font face l'une l'autre.

12. Procédé selon la revendication 11, le fil (53) étant disposé à l'extérieur et à proximité de la première partie concave supérieure et de la première partie concave inférieure des second et premier segments rigides respectifs.

13. Procédé selon l'une quelconque des revendications 11 et 12, le bord supérieur (72) du second segment rigide comprenant en outre une seconde partie concave supérieure qui est disposée sur un côté opposé du bord supérieur (72) à partir de la première partie concave supérieure, et le bord inférieur (82) du premier segment rigide comprenant en outre une seconde partie concave inférieure qui est disposée sur un côté opposé du bord inférieur (82) à partir de la première partie concave inférieure, et
l'alignement d'un second fil (55) à partir de la pointe distale de la partie d'extrémité distale (12) du cathéter allongé (10) et jusqu'à une pointe proximale de la partie d'extrémité proximale (14) du cathéter allongé (10), le second fil (55) étant disposé à l'extérieur de la pluralité de segments rigides (60) et à proximité de la seconde partie concave supérieure et de la seconde partie concave inférieure.

14. Procédé selon la revendication 11, comprenant en outre le positionnement de la pluralité de segments rigides (60) de telle sorte que le bord supérieur (72) du second segment rigide est en contact avec le bord inférieur (82) du premier segment rigide positionné de manière adjacente au second segment rigide et distalement par rapport au second segment rigide.

15. Procédé selon l'une quelconque des revendications 11 à 14, comprenant en outre la thermorétraction de la gaine (20) disposée autour de la pluralité de segments rigides (60).
